# EUROPÄISCHE PATENTSCHRIFT

(11) **EP 2 059 495 B1**
(45) Veröffentlichungstag und Bekanntmachung des Hinweises auf die Patenterteilung: **29.02.2012**
(21) Anmeldenummer: 07801953.6
(22) Anmeldetag: 29.08.2007
(51) Int. Cl.: C07C 41/03, C07C 43/11, C07C 67/26, C07C 213/04, C07D 301/10, C08G 65/26

(54) **VERFAHREN ZUR HERSTELLUNG VON ALKYLENOXIDANLAGERUNGSPRODUKTEN**
METHOD FOR PRODUCING ALKYLENE OXIDE ADDITION PRODUCTS
PROCÉDÉ DE PRÉPARATION DES PRODUITS D'ADDITION D'OXYDES D'ALKYLÈNE

(30) Priorität: 07.09.2006 DE 102006041903
(43) Veröffentlichungstag der Anmeldung: 20.05.2009
(73) Patentinhaber: Cognis IP Management GmbH, 40589 Düsseldorf (DE)
(72) Erfinder: FRANZEN, Stefan, 59174 Kamen (DE); GUTSCHE, Bernhard, 40724 Hilden (DE); FABRY, Bernd, 41352 Korschenbroich (DE); MAHNKE, Eike, Ulf, 42553 Velbert (DE)
(74) Vertreter: Reinhardt, Jürgen
(86) Internationale Anmeldenummer: PCT/EP2007/007531
(87) Internationale Veröffentlichungsnummer: WO 2008/028587

(56) Entgegenhaltungen:
- EP-A- 1 462 446
- EP-A- 1 586 372
- H. KESTENBAUM, A. LANGE DE OLIVEIRA, W. SCHMIDT, F. SCHÜTH, W. EHRFELD, K. GEBAUER, H. LÖWE, T. RICHTER, D. LEBIEDZ ET AL.: "Silver-Catalyzed Oxidation of Ethylene Oxide in a Microreaction System" IND. ENG. CHEM. RES., Bd. 41, 2002, Seiten 710-719, XP002462405 in der Anmeldung erwähnt
- Siegfried Rebsdat; Dieter Mayer: "Ethylene Oxide", Ullmann's Encyclopedia of Industrial Chemistry, 15 March 2001 (2001-03-15), pages 1-27, DOI: 10.1002/14356007.a10_117 Retrieved from the Internet: URL:http://mrw.interscience.wiley.com/emrw /9783527306732/ueic/article/a10_117/curren t/pdf [retrieved on 2010-07-30]

## Beschreibung

### Gebiet der Erfindung

Die Erfindung befindet sich auf dem Gebiet der Herstellung nichtionischer Tenside und betrifft ein neues zweistufiges Verfahren zur integrierten Herstellung von Alkylenoxidanlagerungsprodukte in strukturierten Reaktoren.

### Stand der Technik

Alkylenoxide, wie Ethylen- und Propylenoxid, zählen zu den wichtigsten erdölbasierten Industriechemikalien. Ethylenoxid (EO) ist insbesondere Ausgangsprodukt für die Herstellung von Ethylenglykol, das als Frostschutzmittel beispielsweise dem Flugbenzin zugesetzt wird. Da Ethylen- und Propylenoxid zudem auch mit allen Arten von Stoffen, die über azide Wasserstoffatome verfügen oder nucleophile Zentren beliebiger Form aufweisen, abreagieren, eignet sie sich insbesondere auch zur Anlagerung an Alkohole oder Amine unter Bildung von Polyalkylenglykolketten, die diesen Stoffen einen hydrophilen Charakter verleihen. Der wesentliche Auslass für diese Art von Verbindungen sind nichtionische Tenside, die insbesondere in Waschmitteln und Kosmetika Anwendung finden.

Ethylen- und (in geringerem Umfang) Propylenoxid werden durch Direktoxidation der entsprechenden Alkene an Silberkatalysatoren hergestellt:

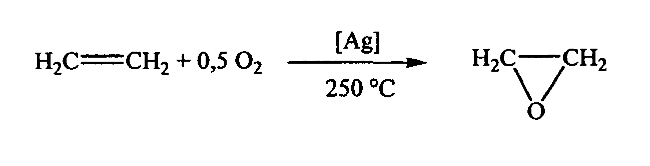

Die Reaktion ist beispielsweise für Ethylenoxid mit 120 kJ/mol exotherm und steht mit der vollständigen Verbrennung des Ethylens zu Kohlendioxid in Konkurrenz, die mit mehr als 1300 kJ/mol wesentlich stärker exotherm verläuft. Die technische Herstellung des Ethylenoxids erfolgt beispielsweise in der Regel in Rohrbündelreaktoren, die bis zu 1000 einzelne Röhren enthalten können und von außen mit einem flüssigen Wärmeträger, wie z.B. Tetralin gekühlt werden, um auch bei sich verstärkender Totaloxidation die Oxidationstemperatur von 230 bis 270 °C einhalten zu können. Der Katalysator, beispielsweise 15 Gew.-% Silber auf Al₂O₃, befindet sich als Schüttung in den Röhren. In der Regel wird der Oxidation mit Sauerstoff der Vorzug gegeben. Nichtsdestotrotz begrenzt man den Umsatz an Ethylen auf etwa 10 bis 15 %, da nur so Selektivitäten von maximal 75 bis 80 % erreicht werden können. Rund ein Viertel des teuren Einsatzmaterials wird also auf diese Weise zu Kohlendioxid verbrannt. Hinzu kommt, dass sich im Endprodukt typisch bis zu 2,5 Vol.-% Wasser und bis zu 10 Vol.-% Kohlendioxid befinden, die vor der Weiterverwertung mit hohem technischem Aufwand abgetrennt werden müssen.

Vor der Umsetzung von Ethylen- bzw. Propylenoxid beispielsweise mit Alkoholen, die zur Bildung der technisch bedeutsamen Niotensidklasse der Alkoholpolyglykolether führt, müssen die Gase einer aufwendigen Reinigung unterworfen werden. Hierzu ist insbesondere eine effektive Trocknung erforderlich, da Wasserspuren zur Bildung von Polyethylenglykolen führt, die als Nebenprodukte äußerst unerwünscht sind. Nachfolgend wird das Kohlendioxid als Kaliumcarbonat gebunden. Das rohe Ethylenoxid wird üblicherweise einer dreistufigen Destillation unterworfen, ehe es den erforderlichen Reinigungsgrad aufweist, wie er für die nachfolgende Alkoxylierungsreaktion bislang erforderlich ist.

Die Alkoxylierung wird üblicherweise diskontinuierlich, beispielsweise in Rührautoklaven oder Schlaufenreaktoren bei Temperaturen zwischen 80 und 200 °C durchgeführt; alternativ kann auch eine Dispersion der flüssigen Reaktionsmischung in eine alkylenoxidhaltige Gasphase erfolgen. Beispielhaft sei dazu auf einen Übersichtsartikel in Chem. Res. 25, 9482-9489 (2005) verwiesen. Typischerweise wird die Verbindung mit dem nucleophilen Zentrum - beispielsweise ein Alkohol, eine Carbonsäure, ein Ester oder ein Amin - zusammen mit dem Katalysator vorgelegt und dann die gewünschte Menge an Alkylenoxid aufgepresst, wobei sich in der Regel abhängig von der Temperatur ein Druck von bis zu 12 bar einstellt. Als Katalysatoren eignen sich basische Verbindungen, wie beispielsweise Alkalialkoholate, oder Lewis-Säuren, wobei letztere den Nachteil aufweisen, dass sie zur Bildung erheblicher Mengen unerwünschter Polyglycolether tendieren.

Aus der EP1586372A1 (Goldschmidt) ist ein Verfahren zur Herstellung von Alkylenoxidanlagerungsprodukten bekannt, bei dem man Nucleophil und Alkylenoxid in der flüssigen Phase in einen Mikroreaktor eindosiert. Die Aufgabe dieser Druckschrift besteht indes darin, die Reaktionsgeschwindigkeit gegenüber konventionellen Verfahren zu verbessern und die Menge an unerwünschten Nebenprodukten in den resultierenden Polyglycolethern zu vermindern. Dies wird dadurch erreicht, dass die Kanäle im Mikroreaktor einen mäanderförmigen Verlauf aufweisen, bei dem sich die Strömungsrichtung ständig umkehrt.

Betrachtet man den Gesamtprozess, dann ist die Herstellung von Alkylenoxidanlagerungsprodukten mit einer ganzen Reihe von technischen und wirtschaftlichen Nachteilen verbunden:
o Im Rahmen der Ethylenoxidherstellung wird ein großer Teil des wertvollen Einsatzstoffes zu wertlosem Kohlendioxid und Wasser verbrannt;
o Die Aufarbeitung des rohen Ethylenoxids ist technisch ausgesprochen aufwendig, jedoch erforderlich, da konventionelle Alkoxylierungsreaktoren eine geringere Reinheit und die Anwesenheit von Verunreinigungen nicht erlauben;
o Die Lagerung und insbesondere der Transport von der Herstellung des Alkylenoxids zum Ort, wo die Alkoxylierung stattfindet, ist wegen der Explosionsgefahr problematisch und ebenfalls mit hohem technischen Schutzaufwand verbunden;
o Die bisherigen Alkoxylierungsverfahren erlauben nur eine diskontinuierliche Herstellung der Alkylenoxidanlagerungsprodukte.

Die Aufgabe der vorliegenden Erfindung hat somit darin bestanden, die genannten Probleme des Stands der Technik durch ein durchgängiges Herstellungsverfahren gleichzeitig zu lösen.

### Beschreibung der Erfindung

Gegenstand der Erfindung ist ein Verfahren zur Herstellung von Alkylenoxidanlagerungsprodukten, welches sich dadurch auszeichnet, dass man
(a) in einem ersten strukturierten Reaktor (auch als "p-Reaktor" bezeichnet) Ethylen und/oder Propylen mit einem Oxidationsmittel in Kontakt bringt und
(b) das dabei gewonnene Ethylenoxid und/oder Propylenoxid gegebenenfalls nach Aufreinigung in einen zweiten strukturierten Reaktor einspeist, in dem es mit einer Verbindung mit nucleophiler Molekülgruppe zur Reaktion gebracht wird.

Das vorgeschlagene neue Verfahren bietet den hohen wirtschaftlichen Vorteil, das Alkylenoxid am gleichen Ort zu erzeugen, an dem auch die Alkoxylierung stattfindet, so dass ein aufwendiger und gefährlicher Transport bzw. Lagerung nicht mehr erforderlich ist. Die Umsetzung der Alkylenoxide im strukturierten Reaktor macht die bisher unvermeidliche destillative Aufreinigung der Alkylenoxide überflüssig. Des Weiteren muss bei der Abtrennung der Alkylenoxide aus dem Alken- und Kohlendioxidreichen Gasstrom auch keine vollständige Reinheit des Ethylen- bzw. Propylenoxids erreicht werden, da die in geringen Konzentrationen verbleibenden Verunreinigungen den Alkoxylierungsprozess nicht stören und auch die Produktqualität nicht negativ beeinflussen. Die Entfernung der leicht siedenden Verunreinigungen aus der EO/PO-Erzeugung erfolgt vielmehr im Anschluss an den Alkoxylierungsprozess im Zuge der ohnehin üblichen Desodorierung. Dies hat den weiteren Vorteil, dass die Siedepunktdifferenzen zwischen Wertprodukt und Verunreinigungen (Kohlendioxid, Ethylen, Formaldehyd, Acetaldehyd) wesentlich größer sind und die Abtrennung daher leichter wird.

### Strukturierte Reaktoren und Mikroreaktionssysteme

Ein zentrales Element der vorliegenden Erfindung besteht in der Erkenntnis, dass strukturierte Reaktoren es ermöglichen, sowohl die Oxidation von Ethylen und Propylen als auch die nachfolgende Alkoxylierung unabhängig von den Explosionsgrenzen durchzuführen, da die Reaktion isotherm geführt werden kann, die Reaktionspartner nur eine minimale Verweilzeit im Reaktor haben und die Reaktionskanäle Durchmesser aufweisen, die die Grenzspaltweite nicht übersteigen. Unter dem Begriff Grenzspaltweite ist dabei der maximale Durchmesser eines Reaktors zu verstehen, bei dem eine durch Explosion entstehende Flamme noch automatisch erlischt. Diese Umstände machen es möglich, beliebige Mischungen von Ethylen bzw. Propylen und Oxidationsmittel bzw. nucleophiler Komponente und Alkylenoxid zu verwenden und trotzdem den Reaktor auch sicher im Explosionsbereich zu betreiben.

Unter dem Begriff "strukturierter Reaktor" ist eine Anordnung von Reaktionskanälen zu verstehen, die einzeln, in Modulen oder auch alle gemeinsam betrieben werden können und sich in einer Matrix befinden, die zur Stabilisierung, Sicherung, Heizung oder Kühlung dient. Eine bevorzugte Ausführungsform eines strukturierten Reaktors stellen Mikroreaktionssystem dar, die auch allgemein als Mikro- oder µ-Reaktoren bezeichnet werden. Ihr Kennzeichen ist es, dass mindestens eine der drei Raumdimensionen des Reaktionsraumes eine Abmessung im Bereich von 1 bis 2000 µm aufweist und die sich damit durch eine hohe übertragungsspezifische innere Oberfläche, kurze Verweilzeiten der Reaktionspartner und hohe spezifische Wärme- und Stofftransportleistungen auszeichnen. Eine ausführliche Darstellung zu diesem Thema findet sich beispielsweise von Jähnisch et al. in Angewandte Chemie Vol. 116, 410-451 (2004). Beispielhaft sei auf die europäische Patentanmeldung EP 0903174A1 (Bayer) verwiesen, in der die Flüssigphasenoxidation organischer Verbindungen in einem Mikroreaktor, bestehend aus einer Schar von parallelen Reaktionskanälen, beschrieben wird. Mikroreaktoren können dabei zusätzlich mikroelektronische Komponenten als integrale Bestandteile enthalten. Im Unterschied zu bekannten mikroanalytischen Systemen besteht bei den Mikroreaktoren keineswegs die Notwendigkeit, dass alle lateralen Dimensionen des Reaktionsraumes im µm-Bereich liegen. Vielmehr werden dessen Abmessungen ausschließlich durch die Art der Reaktion bestimmt. Dementsprechend kommen für bestimmte Reaktionen auch solche Mikroreaktoren in Frage, bei denen eine gewisse Zahl von Mikrokanälen gebündelt wird, so dass Mikro- und Makrokanäle bzw. paralleler Betrieb einer Vielzahl von Mikrokanälen nebeneinander vorliegen können. Vorzugsweise sind die Kanäle parallel zueinander angeordnet, um einen hohen Durchsatz zu ermöglichen und den Druckverlust so gering wie möglich zu halten.

### Träger

Die Träger (auch als "Wafer" bezeichnet), in denen die Struktur und Maße der Mikroreaktionssysteme vorgegeben sind, können Materialkombinationen wie z.B. Silizium-Silizium, Glas-Glas, Metall-Metall, Metall-Kunststoff, Kunststoff-Kunststoff oder Keramik-Keramik oder Kombinationen dieser Materialien darstellen, auch wenn die bevorzugte Ausführungsform ein Silizium-Glas-Verbund, ein Aluminiumoxid oder ein Zeolith ist. Als Träger kommen auch Polyacrylate in Frage, die durch schichtweise Härtung hergestellt werden und besonders kostengünstig in der Herstellung sind. Eine weitere Alternative stellen HAT-Keramiken dar, speziell solche, die von einem druckfesten Mantel umgeben sind sowie Vollmetallreaktoren, bei denen die Reaktionskanäle entsprechend beschichtet sind, um eine Zersetzung des Oxidationsmittels zu verhindern. Die Strukturierung eines beispielsweise 100 bis 2000, vorzugsweise etwa 400 µm dicken Trägers erfolgt vorzugsweise mittels geeigneter Mikrostrukturierungs- bzw. Ätztechniken, z.B. dem reaktivem Ionen-Ätzen ("Reactive Ion Etching"), wodurch z.B. in Silizium dreidimensionale Strukturen unabhängig von der Kristallorientierung gefertigt werden können [vgl. James et al. in Sci. Am. 4, 248 (1993**)]**. In gleicher Weise können z.B. auch Mikroreaktoren aus Glas behandelt werden. Anschließend können dann für die Oxidation bzw. Alkoxylierung übliche Katalysatoren durch geeignete Mikrostrukturierungstechniken, beispielsweise durch Tränken, Imprägnieren, Niederschlagen aus der Gasphase etc. auf die Träger aufgebracht werden.

Auf diese Weise behandelte Träger können 10 bis 1000, vorzugsweise 100 bis 500 und insbesondere 200 bis 300 parallel zueinander verlaufende Mikroreaktionssysteme aufweisen, die entweder parallel oder sequenziell angesteuert und betrieben werden können. Die Geometrie, also der zweidimensionale Verlauf der Kanäle kann dabei sehr unterschiedlich sein: in Frage kommen Geraden, Kurven, Winkel und dergleichen sowie Kombinationen dieser Formelemente. Auch müssen nicht alle Mikroreaktionssysteme die gleiche Geometrie aufweisen. Die Strukturen zeichnen sich dabei durch Abmessungen von 50 bis 1500, vorzugsweise 10 bis 1000 µm und senkrechte Wände aus, wobei die Tiefe der Kanäle 20 bis 1800 und vorzugsweise etwa 200 bis 500 µm beträgt. Die Querschnitte eines jeden Mikroreaktionsraumes, die quadratisch sein können aber nicht müssen, liegen in der Regel in der Größenordnung von 20x20 bis 1500×1500 und insbesondere 100×100 bis 300x300 µm², wie dies beispielsweise auch von Burns et al. in Trans IChemE 77(5), 206 (1999**)** als typisch angegeben wird. Zur Versorgung der Mikroreaktionsräume mit den Edukten wird der Träger an den dafür vorgesehenen Stellen durchgeätzt.

Zum Abschluss wird der strukturierte Träger durch ein geeignetes Verfahren, z. B. anodisches Bonden, mit einem weiteren Träger, z.B. aus Glas, vorzugsweise Pyrex-Glas, verbunden und die einzelnen Strömungskanäle dicht zueinander verschlossen. Selbstverständlich sind abhängig vom Substratmaterial auch andere Aufbau- und Verbindungstechniken zur Realisierung dichter Strömungssysteme möglich, die sich dem Fachmann selbstverständlich erschließen, ohne dass dieser hierzu erfinderisch tätig werden muss.

### Strukturierung der Mikroreaktoren

Die Mikroreaktionssysteme können sich in eine oder mehrere Mischzonen, eine oder mehrere Reaktionszonen, eine oder mehrere Misch- und Reaktionszonen, eine oder mehrere Heiz- und Kühlzonen oder beliebige Kombinationen davon gliedern. Vorzugsweise weisen sie drei Zonen, nämlich zwei Reaktions- und eine Kühlzone auf, wodurch insbesondere zwei-oder mehrstufige Reaktionen in flüssiger oder auch gasförmiger Phase effizient durchgeführt werden können. In der ersten Zone erfolgt dabei die Vermischung zweier Reaktionsteilnehmer sowie deren Reaktion, in der zweiten findet die Reaktion zwischen dem Produkt der ersten Zone und einem weiteren Edukt statt, während in der dritten der Abbruch der Reaktion durch Absenken der Temperatur bewirkt wird. Dabei ist es nicht zwingend erforderlich, die erste und die zweite Reaktionszone thermisch streng voneinander zu trennen. Wenn nämlich die Zugabe eines weiteren Reaktionspartners erforderlich ist oder anstelle eines Mischungspunktes mehrere gewünscht werden, kann dies über die Zone 1 hinaus auch noch in der Reaktionszone 2 stattfinden. Die Mikroreaktionssysteme können dabei sequentiell oder aber gleichzeitig, d.h. parallel mit jeweils definierten Eduktmengen betrieben werden und dabei gleiche oder unterschiedliche Geometrien aufweisen. Eine weitere Möglichkeit, wie sich die Mikroreaktionssysteme in ihrer Geometrie unterscheiden können, besteht im Mischungswinkel, unter dem die Edukte aufeinander treffen und der zwischen 15 und 270° und vorzugsweise 45 bis 180° liegen kann. Des weiteren ist es möglich, jede der drei Zonen unabhängig voneinander zu kühlen oder zu heizen bzw. die Temperatur innerhalb einer Zone beliebig zu variieren, wobei die Reaktionsräume in diesem Beispiel Kanäle darstellen, deren Länge pro Zone 10 bis 500 mm betragen kann.

### Oxidation

Die Herstellung der Alkylenoxide, speziell von Ethylenoxid bzw. Propylenoxid, kann durch direkte Oxidation der entsprechenden Alkene erfolgen. Als Oxidationsmittel kommen für diesen Zweck Sauerstoff oder Luft, aber auch Peroxoverbindungen, wie z.B. Wasserstoffperoxid sowie Ozon in Betracht. In diesem Zusammenhang sei insbesondere auf einen Aufsatz von Schüth et al. in Ind. Eng. Chem. Res 41, 701-719 (2002**)** verwiesen, aus dem der Einsatz von Mikroreaktoren zur silberkatalysierten Oxidation von Ethylen zu Ethylenoxid bekannt ist und dessen Inhalt durch Bezugnahme Bestandteil der vorliegenden Patentanmeldung ist. Es hat sich als besonders wirkungsvoll erwiesen, zur Oxidation der Alkene die Mikroreaktionskanäle entweder mit Silber zu beschichten - das beispielsweise aus der Dampfphase niedergeschlagen werden kann - oder den strukturierten Reaktor direkt aus diesem Metall herzustellen. Als Co-Katalysatoren oder so genannte Promotoren eignen sich Halogenkohlenwasserstoffe, wie z.B. 1,2-Dichlorethan; es ist jedoch auch möglich, das Silber partiell zu halogenieren. Vorzugsweise beträgt die Dicke der Katalysatorschicht im Mittel 50 bis 2.000 und insbesondere 100 bis 1.000 nm.

Die Oxidationsreaktion kann bei Temperaturen im Bereich von 90 bis 300, vorzugsweise 120 bis 280 und insbesondere 180 bis 260 °C durchgeführt werden, wobei in Abhängigkeit vom Oxidationsmittel sowohl unter vermindertem Druck (z.B. Ozon) von 0,1 bar als auch bei Drücken von bis zu 30 bar (Sauerstoff) gearbeitet werden kann. Ein wesentlicher Vorteil besteht, wie schon eingangs erwähnt darin, dass man die Reaktion auch innerhalb der Explosionsgrenzen der Gemische aus Ethylen und/oder Propylen einerseits und Oxidationsmittel andererseits durchführen kann, so dass das Mischungsverhältnis der Einsatzstoffe ausschließlich nach thermodynamischen Gesichtspunkten und nicht unter Beachtung von Sicherheitsrichtlinien gewählt werden kann. Gleichwohl hat es sich bewährt, den Gemischen aus Ethylen und/oder Propylen und Oxidationsmittel weiteres Inertgas, beispielsweise Methan in Anteil von bis zu 70, vorzugsweise bis zu 60 und insbesondere bis zu 50 Vol.-% zuzusetzen.

Wie schon eingangs erwähnt, erlaubt das erfindungsgemäße Verfahren den Einsatz von Ethylen- bzw. Propylenoxid in einer deutlich geringeren Reinheit, als dies bislang in den Verfahren des Stands der Technik erforderlich war. Insbesondere die aufwendige Destillation des Ethylenoxids kann eingespart werden. Die Aufarbeitung erfolgt im Sinne der vorliegenden Erfindung dadurch, dass man den Gasstrom nach Verlassen des ersten und vor Einspeisung in das zweite Mikroreaktionssystem trocknet, um die Bildung von unerwünschtem Glykol in der nachfolgenden Alkoxylierung zu vermeiden. Anschließend werden Ethylen- und/oder Propylenoxid nach Verlassen des ersten Mikroreaktionssystems aus dem Gasstrom auskondensiert und dann in flüssiger Form in das zweite Mikroreaktionssystem einspeist.

### Alkoxylierung

Die Auswahl des Reaktionspartners für die nachfolgende Alkoxylierung ist an sich unkritisch. Bedingung ist lediglich, dass es sich um eine Verbindung mit einem nucleophilen Zentrum, vorzugsweise mit einem aziden Wasserstoffatom handelt. Für diesen Zweck kommen insbesondere Alkohole der Formel (I) in Frage,

R¹OH (I)

in der R¹ für einen linearen oder verzweigten Kohlenwasserstoffrest mit 1 bis 22, vorzugsweise 8 bis 18 Kohlenstoffatomen und 0 oder 1 bis 3 Doppelbindungen steht. Typische Beispiele sind neben den niederen aliphatischen Alkoholen Methanol, Ethanol sowie den isomeren Butanolen und Pentanolen die Fettalkohole, nämlich Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

Eine weitere Gruppe von Verbindungen, die sich als Ausgangsstoffe für die Alkoxylierung eignen, bilden die Carbonsäuren der Formel (II),

R²CO-OH (II)

in der R²CO für einen linearen oder verzweigten Acylrest mit 1 bis 22 Kohlenstoffatomen und 0 oder 1 bis 3 Doppelbindungen steht. Typische Beispiele sind vor allem die Fettsäuren, nämlich Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure. Selbstverständlich können auch funktionalisierte Carbonsäuren, z.B. Hydroxycarbonsäuren wie die Rizinolsäure oder die Zitronensäure oder Dicarbonsäuren wie die Adipin- und Azelainsäure alkoxyliert werden. Anstelle der Säuren können auch die entsprechenden Ester mit C₁-C₂₂ Alkoholen oder Glycerin eingesetzt werden; hier findet dann eine Insertion in die Estergruppe statt.

Schließlich eignen sich als weitere Gruppe von Verbindungen auch Amine der Formel (III) als Ausgangsstoffe,

R³-NH-R⁴ (III)

in der R³ und R⁴ unabhängig voneinander für Wasserstoff, Alkylgruppen mit 1 bis 18 Kohlenstoffatomen oder Hydroxyalkylgruppen mit 1 bis 4 Kohlenstoffatomen stehen. Typische Beispiele sind Methylamin, Dimethylamin, Ethylamin, Diethylamin, Methylethylamin sowie die verschiedenen analog aufgebauten Propyl-, Butyl, Pentyl- und Fettamine.

Die Alkoxylierung findet vorzugsweise in Gegenwart von Katalysatoren statt, die homogener oder heterogener Natur sein können. Bei Einsatz von homogenen Katalysatoren empfiehlt es sich, diese in den Verbindungen mit nucleophilem Zentrum, also beispielsweise in einem Alkohol, zu lösen bzw. zu dispergieren und so dem Mikroreaktionssystem zuzuführen. Als geeignete homogene Katalysatoren seien beispielhaft Alkalihydroxide oder Alkalialkoholate, speziell Kaliumhydroxid, Kalium-tert.butylat und insbesondere Natriummethylat genannt. Werden heterogene Katalysatoren eingesetzt, so werden diese vorzugsweise durch Beschichtung der Kanäle zweiten Mikroreaktionssystems eingesetzt. Vorzugsweise weisen diese Schichten dann im Mittel eine Stärke von 50 bis 2.000 und insbesondere 100 bis 1.000 nm auf. Ein bevorzugtes Beispiel für einen geeigneten Katalysator, den man beispielsweise durch Imprägnieren und nachfolgendes Calcinieren aufbringt, ist das Hydrotalcit. Bei dem Mikroreaktor für die Alkoxylierung handelt es sich vorzugsweise um einen Mikrofallfilmreaktor, wie er beispielsweise in der Druckschrift DE 10036602 A1 (CPC) beschrieben wird; es ist jedoch auch jeder andere Reaktortyp geeignet, der den Kontakt zwischen den Phasen in der dünnen Schicht ermöglicht. In der Regel setzt man das Ethylen- und/oder Propylenoxid und die Verbindungen mit nucleophilem Zentrum im molaren Verhältnis von 1:1 bis 200:1, vorzugsweise 5:1 bis 50:1 und insbesondere 8:1 bis 20:1 ein. Die Reaktionstemperatur kann dabei in Abhängigkeit vom Einsatzmaterial zwischen 50 und 200 °C variieren und liegt vorzugsweise bei 100 bis 120 °C, während die Reaktion sowohl bei Unterdruck, z.B. 0,1 bar als auch bei Drücken bis etwa 12 bar durchgeführt werden kann.

Im Anschluss an die Alkoxylierung findet in der Regel eine Desodorierung statt. Dieser Reaktionsschritt kann dazu genutzt werden, im Ethylen- bzw. Propylenoxid enthaltene Verunreinigungen abzutrennen, um ein in jeder Hinsicht spezifikationsgerechtes und den bisherigen Verfahren des Stands der Technik entsprechendes Produkt zur Verfügung zu stellen.

### Beispiele

### Beispiel 1

Ethylen und Luft wurden in einem Mikrowärmeaustauscher auf eine Temperatur von 220 °C erhitzt. Dabei wurde die Wärme des aus dem Reaktor austretenden Gasgemisches ausgenutzt. In einer Mischeinheit wurden die beiden Gasströme in Kontakt gebracht und dem eigentlichen Reaktor zugeführt. Dieser Teil bestand aus mehreren aufeinander gestapelten Silberfolien, auf die der eigentliche Katalysator aufgebracht war. Der Reaktor wurde bei einem Druck von 2 MPa betrieben. Die Verweilzeit im Reaktor betrug 0,5 Sekunden, in denen ein vollständiger Umsatz des im Feedstrom enthaltenen Sauerstoffs erreicht werden konnte. Die Reaktionswärme wurde über einen Druckwasserkreislauf abgeführt. Das Reaktionsgemisch verließ mit einer Temperatur von 250 °C den Reaktor und bestand zu 73 Mol-% aus dem Inertgas Stickstoff, 3,7 Mol-% nicht umgesetztem Ethylen sowie 4,8 Mol-% des Wertprodukts Ethylenoxid. Aus den Parallelreaktionen waren zusätzlich 16 Mol-% Kohlendioxid, 2 Mol-% Wasser sowie Spuren von Formaldehyd und Acetaldehyd im Gasstrom enthalten. Der Umsatz betrug 61,7 % bei einer Selektivität von 79 %.

Der Gasstrom wurde zweistufig auf eine Temperatur von 90 °C abgekühlt und durch ein Festbett geführt, in dem sowohl das Wasser als auch die Aldehyde adsorptiv gebunden wurden. Der so getrocknete Gasstrom wurde mehrstufig auf 1 MPa entspannt. Dabei wurde der Joule-Thomson-Effekt genutzt, um das Ethylenoxid auszukondensieren. Der Kondensatstrom wurde anschließend in einem Vorlagebehälter gesammelt und enthielt 92,5 Mol-% Ethylenoxid, 7,2 Mol-% Kohlendioxid und kleine Mengen Ethylen. Der Gasstrom wurde einer Aufarbeitung zugeführt, in der CO₂ durch saure Wäsche entfernt wurde, und anschließend in den Reaktor rezykliert.

Zum Nachweis der Anwendbarkeit des so erhaltenen Ethylenoxids in der anschließenden Alkoxylierung wurde ein technisches C_{12/14}-Kokosfettalkoholgemisch (Lorol° Spezial, Cognis Deutschland GmbH & Co. KG) im Durchstromerhitzer auf 130 °C vorgeheizt. In den Rohstoffstrom wurde dann eine 45 Gew.-%ige wässrige Kaliumhydroxidlösung eindosiert, so dass sich eine KOH-Konzentration von ca. 0,1 Gew.-% einstellte. Die so erhaltene Mischung wurde in einem kontinuierlich arbeitenden Mikro-Fallfilmreaktor bei einer Temperatur von ca. 130 °C und einem verminderten Druck von ca. 200 mbar vom Wasser befreit.

Sowohl der getrocknete Feedstrom als auch das Ethylenoxid aus dem Sammelgefäß des vorgeschalteten Oxidationsprozess wurden mit Hilfe von zwei Pumpen auf einen Druck von 30 bar verdichtet und gemeinsam über einen Flüssigkeitsverteiler in einen Mikrorohrbündelreaktor bestehend aus 50 Edelstahlkapillaren mit einer Länge von 25 cm und einem Durchmesser von 1 mm eindosiert. Die bei der Anlagerung des Ethylenoxids an den Alkohol freiwerdende Wärme führte zu einem Anstieg der Temperatur im Reaktor auf 165 bis 180 °C. Aus diesem Grunde wurde der Rohrbündelreaktor mit einem Druckwasserkreislauf gekühlt, mit dessen Hilfe sichergestellt wurde, dass das die Rohrbündel verlassende Alkoxylierungsprodukt nur noch eine Temperatur von 80 °C aufwies. Die über den Kühlwasserkreislauf abgeführte Wärme wurde genutzt, um weiteren Fettalkohol auf die Temperatur von 130 °C vorzuwärmen. Im Anschluss an die Alkoxylierung wurde das Produktgemisch auf 0,15 MPa entspannt und dadurch vorhandene Kohlendioxidreste entfernt. Die anschließende Analyse des Produktes zeigte, dass sich die Anwesenheit des Kohlendioxids nicht negativ auf die Produktqualität ausgewirkt hat. Vielmehr führte die Gegenwart des Kohlendioxids zu einer Inertisierung und damit zur Erhöhung der Sicherheit des untersuchten Alkoxylierungsprozesses.

### Beispiel 2

Ethylen und mit Methan und Argon versetzter Sauerstoff wurden in einem Mikrowärmeaustauscher auf eine Temperatur von 220 °C erhitzt. Dabei wurde die Wärme des aus dem Reaktor austretenden Gasgemisches ausgenutzt. In einer Mischeinheit wurden die beiden Gasströme in Kontakt gebracht und dem eigentlichen Reaktor zugeführt. Dieser Teil bestand aus mehreren aufeinander gestapelten Silberfolien, auf die der eigentliche Katalysator aufgebracht war. Der Reaktor wurde bei einem Druck von 2 MPa betrieben. Die Verweilzeit im Reaktor betrug 0,5 Sekunden, in denen ein vollständiger Umsatz des im Feedstrom enthaltenen Sauerstoffs erreicht werden konnte. Die Reaktionswärme wurde über einen Druckwasserkreislauf abgeführt. Das Reaktionsgemisch verließ mit einer Temperatur von 250 °C den Reaktor und bestand zu 40,2 Mol-% aus den Inertgasen Argon und Methan, 35,3 Mol-% nicht umgesetztem Ethylen sowie 5,0 Mol-% des Wertprodukts Ethylenoxid. Aus den Parallelreaktionen waren zusätzlich 18,1 Mol-% Kohlendioxid, 1,5 Mol-% Wasser sowie Spuren von Formaldehyd und Acetaldehyd im Gasstrom enthalten. Der Umsatz betrug 14,2 % bei einer Selektivität von 86,8 %.

Der Gasstrom wurde zweistufig auf eine Temperatur von 90 °C abgekühlt und durch ein Festbett geführt, in dem sowohl das Wasser als auch die Aldehyde adsorptiv gebunden wurden. Der so getrocknete Gasstrom wurde mehrstufig auf 1 MPa entspannt. Dabei wurde der Joule-Thomson-Effekt genutzt, um das Ethylenoxid auszukondensieren. Der Kondensatstrom wurde anschließend in einem Vorlagebehälter gesammelt und enthielt 92,5 Mol-% Ethylenoxid, 7,2 Mol-% Kohlendioxid und kleine Mengen Ethylen. Der Gasstrom wurde einer Aufarbeitung zugeführt, in der CO₂ durch saure Wäsche entfernt wurde, und anschließend in den Reaktor rezykliert.

Zum Nachweis der Anwendbarkeit des so erhaltenen Ethylenoxids in der anschließenden Alkoxylierung wurde ein technisches C_{12/14}-Kokosfettalkoholgemisch (Lorol° Spezial, Cognis Deutschland GmbH & Co. KG) im Durchstromerhitzer auf 130 °C vorgeheizt. In den Rohstoffstrom wurde dann eine 45 Gew.-%ige wässrige Kaliumhydroxidlösung eindosiert, so dass sich eine KOH-Konzentration von ca. 0,1 Gew.-% einstellte. Die so erhaltene Mischung wurde in einem kontinuierlich arbeitenden Mikro-Fallfilmreaktor bei einer Temperatur von ca. 130 °C und einem verminderten Druck von ca. 200 mbar vom Wasser befreit. Sowohl der getrocknete Feedstrom als auch das Ethylenoxid aus dem Sammelgefäß des vorgeschalteten Oxidationsprozess wurden mit Hilfe von zwei Pumpen auf einen Druck von 30 bar verdichtet und gemeinsam über einen Flüssigkeitsverteiler in einen Mikrorohrbündelreaktor bestehend aus 50 Edelstahlkapillaren mit einer Länge von 25 cm und einem Durchmesser von 1 mm eindosiert. Die bei der Anlagerung des Ethylenoxids an den Alkohol freiwerdende Wärme führte zu einem Anstieg der Temperatur im Reaktor auf 165 bis 180 °C. Aus diesem Grunde wurde der Rohrbündelreaktor mit einem Druckwasserkreislauf gekühlt, mit dessen Hilfe sichergestellt wurde, dass das die Rohrbündel verlassende Alkoxylierungsprodukt nur noch eine Temperatur von 80 °C aufwies. Die über den Kühlwasserkreislauf abgeführte Wärme wurde genutzt, um weiteren Fettalkohol auf die Temperatur von 130 °C vorzuwärmen. Im Anschluss an die Alkoxylierung wurde das Produktgemisch auf 0,15 MPa entspannt und dadurch vorhandene Kohlendioxidreste entfernt. Die anschließende Analyse des Produktes zeigte, dass sich die Anwesenheit des Kohlendioxids nicht negativ auf die Produktqualität ausgewirkt hat. Vielmehr führte die Gegenwart des Kohlendioxids zu einer Inertisierung und damit zur Erhöhung der Sicherheit des untersuchten Alkoxylierungsprozesses.

## Patentansprüche

1. Verfahren zur Herstellung von Alkylenoxidanlagerungsprodukten, **dadurch gekennzeichnet, dass** man
(a) in einem ersten strukturierten Reaktor Ethylen und/oder Propylen mit einem Oxidationsmittel in Kontakt bringt und
(b) das dabei gewonnene Ethylenoxid und/oder Propylenoxid gegebenenfalls nach Aufreinigung in einem zweiten strukturierten Reaktor einspeist, in dem es mit einer Verbindung mit nucleophiler Molekülgruppe zur Reaktion gebracht wird.

2. Verfahren nach Anspruch 1, **dadurch gekennzeichnet, dass** die strukturierten Reaktoren Mikroreaktionssysteme darstellen, bei denen mindestens eine der drei Raumdimensionen des Reaktionsraumes eine Abmessung im Bereich von 1 bis 2000 µm aufweist.

3. Verfahren nach den Ansprüchen 1 und/oder 2, **dadurch gekennzeichnet, dass** die Kanäle im ersten Mikroreaktionssystem mit Silber sowie gegebenenfalls weiteren Co-Katalysatoren beschichtet sind.

4. Verfahren nach mindestens einem der Ansprüche 1 bis 3, **dadurch gekennzeichnet, dass** man als Oxidationsmittel Sauerstoff und/oder Peroxoverbindungen einsetzt.

5. Verfahren nach mindestens einem der Ansprüche 1 bis 4, **dadurch gekennzeichnet, dass** man die Oxidation bei Temperaturen im Bereich von 90 bis 300 °C durchführt.

6. Verfahren nach mindestens einem der Ansprüche 1 bis 5, **dadurch gekennzeichnet, dass** man die Reaktion im Bereich von 0,1 bis 30 bar durchführt.

7. Verfahren nach mindestens einem der Ansprüche 1 bis 6, **dadurch gekennzeichnet, dass** man die Reaktion innerhalb der Explosionsgrenzen der Gemische aus Ethylen und/oder Propylen einerseits und Oxidationsmittel andererseits durchführt.

8. Verfahren nach mindestens einem der Ansprüche 1 bis 7, **dadurch gekennzeichnet, dass** man den Gemischen aus Ethylen und/oder Propylen und Oxidationsmittel weiteres Inertgas zusetzt.

9. Verfahren nach mindestens einem der Ansprüche 1 bis 8, **dadurch gekennzeichnet, dass** man das Ethylen- und/oder Propylenoxid nach Verlassen des ersten Mikroreaktionssystems aus dem Gasstrom auskondensiert und dann in flüssiger Form in das zweite Mikroreaktionssystem einspeist.

10. Verfahren nach mindestens einem der Ansprüche 1 bis 9, **dadurch gekennzeichnet, dass** man als Verbindung mit nucleophilem Zentrum Alkohole der Formel (I) einsetzt,
R¹OH (I)
in der R¹ für einen linearen oder verzweigten Kohlenwasserstoffrest mit 1 bis 22 Kohlenstoffatomen und 0 oder 1 bis 3 Doppelbindungen steht.

11. Verfahren nach mindestens einem der Ansprüche 1 bis 9, **dadurch gekennzeichnet, dass** man als Verbindung mit nucleophilem Zentrum Carbonsäuren der Formel (II) einsetzt,
R²CO-OH (II)
In der R²CO für einen linearen oder verzweigten Acylrest mit 1 bis 22 Kohlenstoffatomen und 0 oder 1 bis 3 Doppelbindungen steht.

12. Verfahren nach mindestens einem der Ansprüche 1 bis 9, **dadurch gekennzeichnet, dass** man als Verbindung mit nucleophilem Zentrum Amine der Formel (III) einsetzt,
R³-NH-R⁴ (III)
In der R³ und R⁴ unabhängig voneinander für Wasserstoff, Alkylgruppen mit 1 bis 18 Kohlenstoffatomen oder Hydroxyalkylgruppen mit 1 bis 4 Kohlenstoffatomen stehen.

13. Verfahren nach mindestens einem der Ansprüche 1 bis 12, **dadurch gekennzeichnet, dass** man die Alkoxylierung in Gegenwart von homogenen oder heterogenen Katalysatoren durchführt.

14. Verfahren nach mindestens einem der Ansprüche 1 bis 13, **dadurch gekennzeichnet, dass** man die Alkoxylierung bei Temperaturen im Bereich von 50 bis 200 °C durchführt.

15. Verfahren nach mindestens einem der Ansprüche 1 bis 14, **dadurch gekennzeichnet, dass** man die Alkoxylierung bei Drücken im Bereich von 0,1 bis 12 bar durchführt.

## Claims

1. Process for preparing alkylene oxide addition products, **characterized in that**
(a) ethylene and/or propylene is contacted with an oxidizing agent in a first structured reactor and
(b) the ethylene oxide and/or propylene oxide obtained, optionally after purification, is fed into a second structured reactor in which it is reacted with a compound with a nucleophilic molecule group.

2. Process according to Claim 1, **characterized in that** the structured reactors are micro reaction systems in which at least one of the three spatial dimensions of the reaction space is within the range from 1 to 2000 µm.

3. Process according to Claim 1 and/or 2, **characterized in that** the channels in the first micro reaction system are coated with silver and optionally further cocatalysts.

4. Process according to at least one of Claims 1 to 3, **characterized in that** the oxidizing agent used is oxygen and/or peroxo compounds.

5. Process according to at least one of Claims 1 to 4, **characterized in that** the oxidation is performed at temperatures within the range from 90 to 300°C.

6. Process according to at least one of Claims 1 to 5, **characterized in that** the reaction is performed within the range from 0.1 to 30 bar.

7. Process according to at least one of Claims 1 to 6, **characterized in that** the reaction is performed within the explosion limits of the mixtures of ethylene and/or propylene on the one hand, and oxidizing agent on the other hand.

8. Process according to at least one of Claims 1 to 7, **characterized in that** further inert gases are added to the mixture of ethylene and/or propylene and oxidizing agent.

9. Process according to at least one of Claims 1 to 8, **characterized in that** ethylene oxide and/or propylene oxide is condensed out of the gas stream after it leaves the first micro reaction system and is then fed into the second micro reaction system in liquid form.

10. Process according to at least one of Claims 1 to 9, **characterized in that** the compounds having nucleophilic sites used are alcohols of the formula (I)
R¹OH (I)
in which R¹ is a linear or branched hydrocarbyl radical having 1 to 22 carbon atoms and 0 or 1 to 3 double bonds.

11. Process according to at least one of Claims 1 to 9, **characterized in that** the compounds having nucleophilic sites used are carboxylic acids of the formula (II)
R²CO-OH (II)
in which R²CO is a linear or branched acyl radical having 1 to 22 carbon atoms and 0 or 1 to 3 double bonds.

12. Process according to at least one of Claims 1 to 9, **characterized in that** the compounds having nucleophilic sites used are amines of the formula (III)
R³-NH-R⁴ (III)
in which R³ and R⁴ are each independently hydrogen, alkyl groups having 1 to 18 carbon atoms or hydroxyalkyl groups having 1 to 4 carbon atoms.

13. Process according to at least one of Claims 1 to 12, **characterized in that** the alkoxylation is performed in the presence of homogeneous or heterogeneous catalysts.

14. Process according to at least one of Claims 1 to 13, **characterized in that** the alkoxylation is performed at temperatures within the range from 50 to 200°C.

15. Process according to at least one of Claims 1 to 14, **characterized in that** the alkoxylation is performed at pressures within the range from 0.1 to 12 bar.

## Revendications

1. Procédé de fabrication de produits d'addition d'oxyde d'alkylène, **caractérisé en ce que**
(a) dans un premier réacteur structuré, de l'éthylène et/ou du propylène sont mis en contact avec un oxydant, et
(b) l'oxyde d'éthylène et/ou l'oxyde de propylène ainsi obtenus sont introduits dans un second réacteur structuré, éventuellement après purification, où ils sont mis en réaction avec un composé contenant un groupe moléculaire nucléophile.

2. Procédé selon la revendication 1, **caractérisé en ce que** les réacteurs structurés sont des systèmes de microréaction, dans lesquels au moins une des trois dimensions de la chambre de réaction présente une dimension dans la plage allant de 1 à 2 000 µm.

3. Procédé selon les revendications 1 et/ou 2, **caractérisé en ce que** les canaux dans le premier système de microréaction sont revêtus avec de l'argent et éventuellement des co-catalyseurs supplémentaires.

4. Procédé selon au moins l'une quelconque des revendications 1 à 3, **caractérisé en ce que** de l'oxygène et/ou des composés peroxo sont utilisés en tant qu'oxydant.

5. Procédé selon au moins l'une quelconque des revendications 1 à 4, **caractérisé en ce que** l'oxydation est réalisée à des températures dans la plage allant de 90 à 300 °C.

6. Procédé selon au moins l'une quelconque des revendications 1 à 5, **caractérisé en ce que** la réaction est réalisée dans la plage allant de 0,1 à 30 bar.

7. Procédé selon au moins l'une quelconque des revendications 1 à 6, **caractérisé en ce que** la réaction est réalisée dans les limites explosives des mélanges d'éthylène et/ou de propylène d'une part et d'oxydant d'autre part.

8. Procédé selon au moins l'une quelconque des revendications 1 à 7, **caractérisé en ce qu'**un gaz inerte est également ajouté aux mélanges d'éthylène et/ou de propylène et d'oxydant.

9. Procédé selon au moins l'une quelconque des revendications 1 à 8, **caractérisé en ce que** l'oxyde d'éthylène et/ou de propylène sont condensés à partir du courant gazeux à la sortie du premier système de microréaction, puis introduits sous forme liquide dans le second système de microréaction.

10. Procédé selon au moins l'une quelconque des revendications 1 à 9, **caractérisé en ce que** des alcools de formule (I) sont utilisés en tant que composé contenant un centre nucléophile,
R¹OH (I)
R¹ représentant un radical hydrocarboné linéaire ou ramifié de 1 à 22 atomes de carbone et 0 ou 1 à 3 doubles liaisons.

11. Procédé selon au moins l'une quelconque des revendications 1 à 9, **caractérisé en ce que** des acides carboxyliques de formule (II) sont utilisés en tant que composé contenant un centre nucléophile
R²CO-OH (II)
R²CO représentant un radical acyle linéaire ou ramifié de 1 à 22 atomes de carbone et 0 ou 1 à 3 doubles liaisons.

12. Procédé selon au moins l'une quelconque des revendications 1 à 9, **caractérisé en ce que** des amines de formule (III) sont utilisées en tant que composé contenant un centre nucléophile
R³-NH-R⁴ (III)
R³ et R⁴ représentant indépendamment l'un de l'autre l'hydrogène, des groupes alkyle de 1 à 18 atomes de carbone ou des groupes hydroxyalkyle de 1 à 4 atomes de carbone.

13. Procédé selon au moins l'une quelconque des revendications 1 à 12, **caractérisé en ce que** l'alcoxylation est réalisée en présence de catalyseurs homogènes ou hétérogènes.

14. Procédé selon au moins l'une quelconque des revendications 1 à 13, **caractérisé en ce que** l'alcoxylation est réalisée à des températures dans la plage allant de 50 à 200 °C.

15. Procédé selon au moins l'une quelconque des revendications 1 à 14, **caractérisé en ce que** l'alcoxylation est réalisée à des pressions dans la plage allant de 0,1 à 12 bar.
